# FASCICULE DE BREVET EUROPEEN

(11) **EP 0 387 314 B1**
(45) Date de publication et mention de la délivrance du brevet: **04.05.1994**
(21) Numéro de dépôt: 89907642.6
(22) Date de dépôt: 07.07.1989
(51) Int. Cl.: A61K 31/655

(54) **UTILISATION D'AGENT ACTIF CONTRE LES RETROVIRUS ET PRODUITS AINSI MIS EN UVRE**
ANWENDUNG VON GEGEN RETROVIREN AKTIVEN WIRKSTOFFEN UND SO ERHALTENE PRODUKTE
USE OF AN AGENT ACTIVE AGAINST RETROVIRUS AND PRODUCTS SO PRODUCED

(30) Priorité: 26.08.1988 EP 88870139
(43) Date de publication de la demande: 19.09.1990
(73) Titulaire: Vandevelde, Michel, B-1301 Bierges (BE); Margery, Hélène, B-1301 Bierges (BE)
(72) Inventeur: Vandevelde, Michel, B-1301 Bierges (BE); Margery, Hélène, B-1301 Bierges (BE)
(74) Mandataire: Claeys, Pierre
(86) Numéro de dépôt international: BE8900033
(87) Numéro de publication internationale: WO9001935

(56) Documents cités:
- EP-A- 0 196 185
- EP-A- 0 240 098
- EP-A- 0 285 357
- EP-A- 0 307 914
- FR-A- 2 612 515
- Chemical Abstracts, volume 107, No. 21, 23 Nov. 1987, (Columbus, 0hio, US), K.D. Jentsch et. al., see pages 22, 23
- Chemical Abstracts, volume 104, No 23, 9 June 1986 (Columbus, 0hio, US), J. Balzarini et al.: see page 42
- Le Monde du Jeudi 5 mars 1992, p. 9
- JAMA, Sept. 11, 1987, vol. 258, no.10, p. 1347-1351
- Martindale 29ieme édition, 1989, p. 679
- The Pharmacological Basis of Therapeutics, 4ieme edition, p.1144

## Description

La présente invention concerne un procédé pour rendre un produit à application topique sur la peau, les muqueuses et les sécrétions corporelles, inhibiteur de la propagation des virus du groupe rétrovirus, en particulier des virus d'immunodéficience humaine VIH. Elle concerne également les produits destinés à une application topique sur la peau, les muqueuses ou des sécrétions corporelles.

Depuis la détermination relativement récente du syndrome d'immunodéficience humaine, maladie appelée sida, beaucoup de recherches ont été effectuées sur ce sujet. On a pu établir l'existence d'une série de virus, faisant partie du groupe des rétrovirus et parmi lesquels on peut citer les virus lymphotropiques de cellule T humaine, appelés virus VIH.

Il a pu être démontré que ces virus étaient présents, chez les porteurs, dans la plupart des liquides intérieurs, notamment dans le sang, la salive, le sperme, la lymphe, le lait, les larmes, les sécrétions vaginales (v. D. ZAGURY et cons., Science, oct. 1984, vol. 226, n° 4673, p. 449-451; R. ZITTOUN, Syndrome Immuno Déficitaire Acquis, Doins Editeurs, Paris, 1986, p. 183).

Si, jusqu'à présent, il n'a pas pu être possible de mettre au point un vaccin ou un médicament efficace contre le sida, il est devenu petit à petit possible d'émettre quelques interprétations à propos du développement de ces virus. Il pourrait exister une inoculation par contact, c'est-à-dire à travers une muqueuse ou la peau; les virus inoculés sembleraient alors connaître une phase de réplication pendant laquelle les particules restent dans la sphère de la muqueuse ou la peau du porteur. Cette phase pourrait se prolonger pendant plusieurs mois. Ce n'est que dans une seconde phase que les virus et/ou ses constituants dissémineraient à partir de la muqueuse (R. ZITTOUN, op. cit., p. 184).

La reproduction des rétrovirus est permise par la présence d'une enzyme. la réverse-transcriptase. Il est connu que la suramine est un inhibiteur de la réverse-transcriptase d'un certain nombre de rétrovirus. Des essais in vitro ont par ailleurs montré l'efficacité de certaines molécules, notamment de composés disazoïques et de la suramine, dans l'inhibition de la réverse-transcriptase du virus VIH-III (H. MITSUYA et cons., Science, octobre 1984, Vol. 226, n° 4671, p. 172 à 174; E. DE CLERCQ, Cancer Letters, 8 (1979), 9-22).

On a en outre déjà décrit qu'une solution de 50 µg /ml de suramine a in vitro un effet de protection contre les actions cytopathiques du virus VIH (J. BALZARINI et cons., Comparative inhibitory effects of suramine..., Int. J. Cancer 37, 451-457 (1986)).

Des essais ultérieurs ont été effectués in vivo sur des porteurs du virus du sida. On leur a administré par voie orale des doses d'inhibiteurs de la réverse-transcriptase, et notamment de suramine. Si le virus ne pouvait plus être décelé, pendant le traitement systémique, dans des prélèvements effectués sur des patients infectés, il s'est manifesté à nouveau peu après la cessation du traitement (S. BRODER et cons., The Lancet, septembre 21, 1985, p. 627 à 630; T.R. HARRISSON, Principes de médecine interne, 4ème éd. française, Paris, 1988, p. 1395). Cette voie semble donc actuellement abandonnée.

La présente invention a pour but de proposer des moyens prophylactiques dans la lutte contre le sida qui, en outre, soient de préférence aisés à préparer et d'un coût modéré. Au contraire de la tendance actuelle des spécialistes, décrite ci-dessus, elle propose de mettre en valeur, d'une part, la connaissance de l'activité inhibitrice, in vitro, de composés disazoïques sur la réverse-transcriptase du VIH et, d'autre part, l'interprétation actuelle d'un développement en deux phases du virus lors d'une infection par contact. L'invention a pour objet de rendre défavorables à la transmission, à la propagation et à la reproduction du virus les produits, qui sont susceptibles, pour une raison quelconque, d'entrer en contact avec la peau, les muqueuses ou des sécrétions corporelles. Il s'agit donc d'une mesure préventive, antérieure à toute application sur le corps humain ou animal.

Pour résoudre ce problème, l'invention prévoit un procédé présentant les particularités de le revendication 1. Comme agent actif, on utilise avantageusement un agent d'inhibition de la réverse-transcriptase des rétrovirus. Il est évident qu'on peut estimer utilisable suivant l'invention n'importe quel agent actif, quel que soit son mode d'action pour combattre les virus visés, et donc également si celui-ci ne consiste pas en l'inhibition de la réverse-transcriptase. On peut par exemple imaginer un agent inhibant la liaison spécifique du virus à la molécule T4 des lymphocytes.

Suivant un mode de réalisation de l'invention, au moins un des agents d'inhibition est un composé disazoïque. De préférence, un agent d'inhibition est un sel hexasodique d'acide 8,8'-[carbonyl-bis-[imino-3,1-phénylènecarbonylimino-(4-méthyl-3,1-phénylène)-carbonylimino]]-bis-1,3,5-naphtalènetrisulfonique. Cette molécule est appelée également suramine sodique, germanine, 205 Bayer, 309 Fourneau, moranyl, antrypol, naganol ou naganine. Cette molécule est connue depuis longtemps, notamment pour le traitement de la trypanosomiase ou de l'onchocercose. Comme on l'a dit précédemment, sa valeur comme agent d'inhibition de la réverse-transcriptase de rétrovirus était connue depuis longtemps et on vient de montrer son activité in vitro contre la réverse-transcriptase du VIH III. Cette substance, qui est un dérivé de la benzidine, se présente sous la forme d'une poudre blanche, aisément soluble dans l'eau, sa toxicité est peu élevée, et son coût de fabrication est modéré.

On envisage également suivant l'invention, comme autres composés disazoïques, entre autres la benzidine, la dianilidurée, le pyridium, la néotropine, le rouge congo, le bleu de trypan, le rouge de trypan, le violet de trypan. En ce qui concerne des composés apparentés à la suramine, dont l'action d'inhibition a éte examinée in vitro, on peut en trouver une liste par exemple dans J. BALZARINI et cons., Comparative inhibitory effects of suramin..., Int. J. Cancer, 37, 451-457 (1986), et K.D. JENTSCH, Inhibition of Human immunodeficiency virus type I reverse transcriptase..., J. Gen. Virol., 1987, 68(8), 2183-92.

Il est connu par ailleurs que les virus VIH pénètrent dans les cellules des lymphocytes T4 d'un être humain infecté et que l'ARN viral, après diverses opérations, intègre le génome du lymphocyte T4 attaqué.

En présence d'agents pathogènes ou d'un contact allogénique par exposition par exemple à du sperme ou du sérum, il y a une réaction du corps par activation de ses cellules lymphocytaires. Cette activation a pour effet, en plus de la mort de la cellule hòte, une réplication du virus qui va infecter des cellules voisines, et ainsi de suite.

Un porteur de virus du sida a donc tout avantage à suivre des habitudes de vie hygiéniques qui écartent au maximum un danger d'activation des cellules porteuses, et donc une réaction immunitaire de son organisme.

On a pour cette raison prévu suivant l'invention d'introduire dans les produits susceptibles d'entrer localement en contact avec la peau, les muqueuses ou des sécrétions corporelles, conjointement à l'agent actif utilisé suivant l'invention, un agent antiseptique approprié pour une application locale. Comme agent antiseptique de ce genre, on peut envisager notamment ceux capables de libérer du chlore. La chlorhexidine (1,6-di-(4-chlorophényldiguanido)hexane) en est un exemple préféré étant donné sa très faible toxicité et la disponibilité aisée de cette substance peu coûteuse (v. N. SENIOR, Some observations on the formulation and properties of chlorhexidine, J. Soc. Cosmet. Chem., 24, 259-278 (1973)).

D'autres modes de réalisation du procédé suivant l'invention ressortiront des revendications 1 à 7.

L'invention prévoit également des produits destinés à une application topique sur la peau, les muqueuses ou des sécrétions corporelles, ces produits présentant les particularités de la revendication 8. On utilisera avantageusement de la suramine sodique. La quantité minimale de suramine sodique permettant une efficacité maximale contre les virus mêmes est de 250 µg par ml de solution appliquée.

D'autres formes de réalisation avantageuses de l'invention ressortent notamment des sous-revendications 8 à 21.

Les produits suivant l'invention peuvent donc être des supports solides, par exemple des supports fibreux comme des mouchoirs, des serviettes, des bandages, des pansements, de l'ouate, ou des supports en forme de film, à base de matière plastique ou caoutchouteuse, comme des gants de médecin ou de dentiste, des préservatifs ou diaphragmes, des produits d'emballage.

Les produits suivant l'invention forment en fait un milieu défavorable à la transmission du virus à une personne saine, en réalisant une espèce de barrière protectrice. Suivant une forme de réalisation perfectionnée, les produits suivant l'invention contiennent en outre un antiseptique et ils représentent ainsi simultanément une barrière protectrice pour le porteur de virus lui aussi. Il se protège ainsi contre tout agent pathogène venant de l'extérieur, qui risquerait d'activer son système immunitaire.

Les produits suivant l'invention peuvent aussi comporter un excipient pâteux ou un véhicule liquide. Par la présence d'au moins un agent suivant l'invention dans les produits cosmétiques ou d'hygiène journalière, comme les dentifrices, ces produits sont, préalablement à leur application, rendus incapables de transmettre le virus, précisément là où l'infection par celui-ci peut se propager aisément. Secondairement, ils peuvent avoir un effet thérapeutique local sur des virus éventuellement présents à l'endroit de l'application du produit. On peut même prévoir aussi un traitement préalable comparable de produits thérapeutiques quelconques, à application locale, par exemple par un agent inhibiteur de la réverse-transcriptase des VIH.

L'invention va à présent être illustrée à l'aide de quelques exemples de réalisation.

### Exemple 1

A 100 g d'une pâte dentifrice comprenant 4 % de ricinilate, on mélange 10 mg de suramine sodique.

### Exemple 2

Composition d'un bain de bouche :

| | |
|---|---|
| perborate de sodium | 8 g |
| borax | 32 g |
| chlorure de sodium | 20 g |
| bicarbonate de sodium | 40 g |
| suramine sodique | 10 mg à 2 g |
| essence de menthe | 3 gouttes |

1 cuillère à café dans une tasse d'eau tiède.

### Exemple 3

Produit à pulvériser dans la bouche :

| | |
|---|---|
| suramine sodique | 1 mg à 100 mg |
| alcoolature de citron PBV | 1 goutte |
| eau ad | 10 ml |
| flacon nébulisateur. | |

### Exemple 4

Savon solide :

| | |
|---|---|
| suramine sodique | 1 g à 5 g |
| savon potassique | 74 g |
| pour 1 brique. | |

### Exemple 5

Savon liquide :

| | |
|---|---|
| suramine sodique | 25 mg à 1,5 g |
| savon potassique | 60 g |
| essence de lavande | 10 gouttes |
| solution antiseptique ad | 100 g |
| pour 100 g de savon. | |

### Exemple 6

Shampoing :

| | |
|---|---|
| dianilidurée | 1 g à 20 g |
| huile de ricin | 2 g |
| lécithine | 2 g |
| acide undécylique | 4 g |
| Tensomel® G 211 | 4 g |
| propylèneglycol | 20 g |
| isopropanol | 20 g |
| Tensergex® SP DL 6 | 100 g |
| eau ad | 200 ml |
| pour 200 ml de shampooing. | |

On dissout la lécithine et l'acide undécylique dans le mélange d'isopropanol et d'huile de ricin, puis on ajoute le propylèneglycol et les autres composants. Le produit est à agiter avant l'emploi.

### Exemple 7

Lotion capillaire :

| | |
|---|---|
| suranime sodique | 10mg à 1,5 g |
| nicotinate de benzyle | 25 mg |
| dexpanthoténol | 3 g |
| Teinture de jaborandi | 5 g |
| éthanol | 50 g |
| essence de lavande | 1 g |
| Eau ad | 150 ml |
| pour 150 ml de lotion. | |

### Exemple 8

Lotion après rasage :

| | |
|---|---|
| suramine sodique | 10 mg à 1 g |
| chlorhydrate de diphénydramine | 1 g |
| oxyde de zinc | 8 g |
| liniment au veegum suivant FMS ad | 100 ml |
| pour 100 ml de lotion. | |

### Exemple 9

Crème :

| | |
|---|---|
| suramine sodique | 10 mg à 1 g |
| triéthanolamine | 1 g |
| glycérol | 2,5 g |
| cire blanche | 2,5 g |
| acide stéarique | 6 g |
| huile d'amande | 7,5 g |
| essence de lavande | 2 gouttes |
| aqua conservans ad | 50 g FMS |
| pour 50 g de crème. | |

### Exemple 10

Talc : dose minimale inhibitrice = 100 µg par ml

| | |
|---|---|
| bleu de trypan | 5 mg à 1,0 g |
| essence de lavande | 5 gouttes |
| talc ad | 100 g |
| pour 100 g de talc. | |

### Exemple 11

Poudre pour gant de chirurgien :

| | |
|---|---|
| suramine sodique | 10 mg à 2 g |
| amylase amylopectine suivant USPC ad | 100 g |

pour fournir une poudre de poudrage absorbable.

Il faut noter que cette poudre peut par exemple se trouver entre deux pellicules, par exemple caoutchouteuses, et qu'elle peut ne pas être elle-même en contact direct avec la peau.

### Exemple 12

Condom :
D'une manière courante, un condom en matière caoutchouteuse est lubrifié d'une vaseline renfermant de la suramine sodique :

| | |
|---|---|
| suramine sodique | 10 mg à 2 g |
| vaseline ad | 50 g |
| pour 50 g de produit. | |

### Exemple 13

Papier de toilette, bandes et tampons hygiéniques, ouate, etc.

On prépare tout d'abord une poudre à pulvériser dont la composition est par exemple la suivante :

| | |
|---|---|
| suramine sodique | 10 g |
| sous-gallate de bismuth | 50 g |
| peroxyde de zinc | 100 g |
| talc | 840 g |
| pour 1 kg de poudre à pulvériser. | |

On pulvérise ensuite d'une manière courante cette poudre qui adhère aux fibres des produits traités.

### Exemple 14

Solution aqueuse à pulvériser.

1g de suramine sodique est dissous dans 10g d'huile de silicone ou dans 2g de lécithine. Le tout est émulsionné à l'aide de 30g de Cetomacrogol 1000 ou de Tween® 60 ou 80, selon les besoins. On ajoute de l'eau pour obtenir 1l de solution. On ajoute aussi 1 % de chlorhexidine par rapport à la composition globale. Si nécessaire, on ajoute encore 5 gouttes d'essence de rose ou de chlorophylle par exemple.

### Exemple 15

Poudre pour gants de chirurgien ou pansements.

Pour fabriquer 1kg de poudre, on mélange à de l'amidon de maïs stérilisable et résorbable 1g de suramine sodique et 0,5% de chlorhexidine.

### Exemple 16

Solution huileuse pour des gouttes auriculaires, le traitement des gencives, des bains de bouche, etc...

| | |
|---|---|
| Huile de silicone | 100 g |
| Chlorhexidine | 1 g |
| Suramine sodique | 1 g |

Préparation sous forme émulsionnée.

On peut par exemple envisager que l'agent d'inhibition soit fixé de manière libérable sur un support solide par d'autres moyens que ceux décrits, comme par exemple par imprégnation.

On peut prévoir qu'on découvre un jour un agent actif pour combattre les virus du groupe des rétrovirus, qui simultanément présente une action antiseptique vis-à-vis des agents pathogènes extérieurs. L'invention s'étend évidemment à son utilisation dans des produits susceptibles d'entrer localement en contact avec la peau.

## Revendications

1. Procédé pour rendre un produit à application topique sur la peau, les muqueuses ou les sécrétions corporelles, inhibiteur de la propagation des virus du groupe rétrovirus, en particulier les virus d'immunodéficience humaine VIH, comprenant l'addition à ce produit d'un agent actif choisi parmi le groupe comprenant la benzidine, la dianilidurée, la suramine, le pyridium, la néotropine et des composés disazoïques aromatiques, tels que le rouge Congo, le bleu de trypan, le rouge de trypan et le violet de trypan.

2. Procédé suivant la revendication 1, caractérisé en ce que, comme agent actif, on utilise un agent inhibant la liaison du virus à une molécule T₄ de lymphocyte.

3. Procédé suivant l'une ou l'autre des revendications 1 et 2, caractérisé en ce qu'il comprend l'addition de sel hexasodique d'acide 8,8'-[carbonyl-bis-[imino-3,1-phénylène carbonylimino-(4-méthyl-3,1-phénylène)-carbonyl-imino]]-bis-1,3,5-naphtalènetrisulfonique.

4. Procédé suivant l'une quelconque des revendications 1 à 3, caractérisé en ce qu'il comprend la mise en oeuvre conjointe d'au moins un agent antiseptique approprié pour une application topique.

5. Procédé suivant l'une quelconque des revendications 1 à 4, caractérisé en ce que l'agent actif est de la suramine à une concentration d'au moins 70 µg/ml.

6. Procédé suivant la revendication 5, caractérisé en ce que l'agent actif est de la suramine à une concentration de 75 à 250 µg/ml.

7. Procédé suivant la revendication 6, caractérisé en ce que l'agent actif est de la suramine à une concentration de 100 à 200 µg/ml.

8. Produits destinés à une application topique sur la peau, les muqueuses ou les sécrétions corporelles, comprenant un agent actif inhibitant la propagation des virus du groupe rétrovirus, en particulier des virus d'immunodéficience humaine VIH, choisi parmi le groupe comprenant la benzidine, la dianilidurée, la suramine, le pyridium, la néotropine et des composés disazoïques aromatiques, tels que le rouge Congo, le bleu de trypan, le rouge de trypan et le violet de trypan.

9. Produit suivant la revendication 8, caractérisé en ce qu'il comprend, comme agent susdit, un agent inhibant la liaison du virus à une molécule T₄ de lymphocyte.

10. Produit suivant l'une ou l'autre des revendications 8 et 9, caractérisé en ce qu'il comprend, comme agent susdit, du sel hexasodique d'acide 8,8'-[carbonyl-bis-[imino-3,1-phénylène carbonylimino-(4-méthyl-3,1-phénylène)-carbonyl-imino]]-bis-1,3,5-naphtalènetrisulfonique.

11. Produit suivant l'une quelconque des revendications 8 à 10, caractérisé en ce qu'il comprend en outre au moins un agent antiseptique approprié pour une application topique.

12. Produit suivant l'une quelconque des revendications 8 à 11, caractérisé en ce qu'il comprend, comme agent actif, de la suramine à une concentration d'au moins 70 µg/ml.

13. Produit suivant la revendication 12, caractérisé en ce qu'il comprend, comme agent actif, de la suramine à une concentration de 75 à 250 µg/ml.

14. Produit suivant la revendication 13, caractérisé en ce qu'il comprend, comme agent actif, de la suramine à une concentration de 100 à 200 µg/ml.

15. Produit suivant l'une quelconque des revendications 8 à 14, à mettre en oeuvre comme composition ou produit cosmétique, hygiénique ou dentifrice.

16. Produit suivant l'une quelconque des revendications 8 à 15, caractérisé en ce qu'il comprend une matière de support solide sur laquelle sont supportés par un procédé approprié quelconque le ou les agents actifs susdits.

17. Produit suivant la revendication 16, caractérisé en ce qu'il consiste en mouchoirs, serviettes, bandages, pansements, gants, produits d'emballage, préservatifs, diaphragmes, ouate, papier hygiénique et moyens analogues.

18. Produit suivant l'une quelconque des revendications 8 à 15, caractérisé en ce qu'il comprend une pâte, dans laquelle le ou les agents actifs susdits sont mélangés.

19. Produit suivant la revendication 18, caractérisé en ce qu'il consiste en une pâte dentifrice, un onguent, une crème, un shampoing, un fard.

20. Produit suivant l'une quelconque des revendications 8 à 15, caractérisé en ce qu'il comprend un véhicule liquide dans lequel le ou les agents actifs susdits sont en solution ou en suspension.

21. Produit suivant la revendication 20, caractérisé en ce qu'il consiste en un bain de bouche, une solution à pulvériser, un désodorisant, une lotion.

22. Utilisation d'un agent actif choisi parmi le groupe comprenant la benzidine, la dianilidurée, la suramine, le pyridium, la néotropine et des composés disazoïques aromatiques, tels que le rouge Congo, le bleu de trypan, le rouge de trypan et le violet de trypan, pour la fabrication d'un produit pharmaceutique prophylactique, destiné à entrer localement en contact avec la peau, les muqueuses ou les sécrétions corporelles et à inhiber à cet endroit la propagation des virus du groupe rétrovirus, en particulier des virus d'immunodéficience humaine VIH.

## Claims

1. A method of making a product to be topically applied on the skin, mucosas or body secretions inhibitive to the spread of the viruses of the retrovirus group, particularly the human immunodeficiency viruses HIV, which comprises adding to this product an active agent selected from the group consisting of benzidine, dianilidurea, suramin, pyridium, neotropin and aromatic diazoïc compounds, such as Congo red, trypane blue, trypane red and trypane violet.

2. A method according to claim 1, wherein an agent inhibiting the binding of the virus to a molecule T4 of lymphocyte is used as active agent.

3. A method according to anyone of claims 1 or 2, comprising adding a hexasodium salt of 8.8'-[carbonyl-bis-[imino-3.1-phenylene-carbonylimino-(4-methyl-3.1-phenylene)-carbonylimino]]-bis-1.3.5-naphthalenetrisulfonic acid.

4. A method according to anyone of claims 1 to comprising a simultaneous addition of at least one antiseptic agent appropriate for a topical application.

5. A method according to anyone of claims 1 to 4 wherein said active agent is suramin at a concentration of at least 70 µg/ml.

6. A method according to claim 5, wherein said active agent is suramin at a concentration of 75 to 250 µg/ml.

7. A method according to claim 6, wherein the active agent is suramin at a concentration of 100 to 200 µg/ml.

8. Products to be topically applied on the skin, mucosas or body secretions, comprising an active agent inhibiting the spread of the viruses of the retrovirus group, particularly of the human immunodeficiency viruses HIV, selected from the group consisting of benzidine, dianilidurea, suramin, pyridium, neotropin and aromatic diazoïc compounds, such as Congo red, trypane blue, trypane red and trypane violet.

9. A product according to claim 8, comprising, as active agent, an agent inhibiting the binding of the virus to a molecule T4 of lymphocyte.

10. A product according to anyone of claims 8 and 9 comprising, as active agent, a hexasodium salt of 8.8'-[carbonyl-bis-[imino-3.1-phenylene-carbonylimino-(4-methyl-3.1-phenylene)-carbonylimino]]-bis-1.3.5-naphthalenetrisulfonic acid.

11. A product according to anyone of claims 8 to 10, further comprising at least one antiseptic agent appropriate for topical application.

12. A product according to anyone of claims 8 to 11, comprising, as active agent, suramin at a concentration of at least 70 µg/ml.

13. A product according to claim 12, cromprising, as active agent, suramin at a concentration of 75 to 250 µg/ml.

14. A product according to claim 13, comprising, as active agent, suramin at a concentration of 100 to 200 µg/ml.

15. A product according to anyone of claims 8 to 14, which is to use in the form of cosmetic or hygienic composition or product or of toothpaste.

16. A product according to anyone of claims 8 to 15, which comprises a solid support material on which the active agent(s) is (are) supported by any appropriate process.

17. A product according to claim 16, which consists of handkerchieves, towels, bandagings, dressings, gloves, packaging products, contraceptive sheathes, diaphragms, cotton wool or toilet paper and analoguous means.

18. A product according to anyone of claims 8 to 15, which comprises a paste, wherein the active agent(s) is (are) mixed.

19. A product according to claim 18, which consists of a toothpaste, an ointment, a cream, a shampoo, a make-up.

20. A product according to anyone of claims 8 to 15, which comprises a liquid vehicle wherein the active agent(s) is (are) dissolved or suspended.

21. A product according to claim 20, which consists of a mouthwash, a solution to be sprayed, a deodorant, a lotion.

22. Use of an active agent selected from the group consisting of benzidine, dianilidurea, suramin, pyridium, neotropin and aromatic diazoïc compounds, such as Congo red, trypane blue, trypane red and trypane violet, for manufacturing a prophylactic pharmaceutical product, intended to come into topical contact with the skin, mucosas or body secretions and to inhibate there the spread of viruses of the retrovirus group, particularly the human immunodeficiency viruses HIV.

## Patentansprüche

1. Verfahren zur Herstellung eines Produktes zur topischen Anwendung auf der Haut, den Schleimhäuten oder Körperabsonderungen, das die Ausbreitung eines Virus aus der Gruppe der Retroviren hemmt, insbesondere des menschlichen Immunschwächevirus HIV, umfassend die Zugabe eines Wirkstoffes zu diesem Produkt, ausgewählt aus Benzidin, Dianilidharnstoff, Suramin, Pyridium, Neotropin und aromatischen Diazoverbindungen,wie Kongorot, Trypanblau, Trypanrot und Trypanviolett.

2. Verfahren nach Anspruch 1, dadurch gekennzeichnet, daß man als Wirkstoff einen Stoff verwendet, der die Bindung des Virus an ein T₄-Lymphocytenmolekül hemmt.

3. Verfahren nach einem der Ansprüche 1 oder 2, dadurch gekennzeichnet, daß es die Zugabe des Hexanatriumsalzes der 8,8'-[Carbonyl-bis-[imino-3,1-phenylencarbonylimino-(4-methyl-3,1-phenylen)-carbonyl-imino]]-bis-1,3,5-naphthalintrisulfonsäure umfaßt.

4. Verfahren nach einem der Ansprüche 1 bis 3, dadurch gekennzeichnet, daß es die gemeinsame Verwendung mit mindestens einem antiseptischen Mittel umfaßt, das für eine topische Anwendung geeignet ist.

5. Verfahren nach einem der Ansprüche 1 bis 4, dadurch gekennzeichnet, daß der Wirkstoff Suramin in einer Konzentration von mindestens 70 µg/ml ist.

6. Verfahren nach Anspruch 5, dadurch gekennzeichnet, daß der Wirkstoff Suramin in einer Konzentration von 75 bis 250 µg/ml ist.

7. Verfahren nach Anspruch 6, dadurch gekennzeichnet, daß der Wirkstoff Suramin in einer Konzentration von 100 bis 200 µg/ml ist.

8. Produkte für die topische Anwendung auf der Haut, den Schleimhäuten oder Körperabsonderungen, umfassend einen Wirkstoff, der die Ausbreitung eines Virus aus der Gruppe der Retroviren hemmt, insbesondere des menschlichen Immunschwächevirus HIV, ausgewählt aus Benzidin, Dianilidharnstoff, Suramin, Pyridium, Neotropin und aromatischen Diazoverbindungen, wie Kongorot, Trypanblau, Trypanrot und Trypanviolett.

9. Produkt nach Anspruch 8, dadurch gekennzeichnet, daß es als den vorgenannten Stoff einen Stoff umfaßt, der die Bindung des Virus an ein T₄-Lymphocytenmolekül hemmt.

10. Produkt nach einem der Ansprüche 8 oder 9, dadurch gekennzeichnet, daß es als vorgenannten Stoff Hexanatriumsalz der 8,8'-[Carbonyl-bis-[imino-3,1-phenylencarbonylimino-(4-methyl-3,1-phenylen)-carbonyl-imino]]-bis-1,3,5-naphthalintrisulfonsäure umfaßt.

11. Produkt nach einem der Ansprüche 8 bis 10, dadurch gekennzeichnet, daß es außerdem mindestens ein antiseptisches Mittel umfaßt, das für eine topische Anwendung geeignet ist.

12. Produkt nach einem der Ansprüche 8 bis 11, dadurch gekennzeichnet, daß es als Wirkstoff Suramin in einer Konzentration von mindestens 70 µg/ml umfaßt.

13. Produkt nach Anspruch 12, dadurch gekennzeichnet, daß es als Wirkstoff Suramin in einer Konzentration von 75 bis 250 µg/ml umfaßt.

14. Produkt nach Anspruch 13, dadurch gekennzeichnet, daß es als Wirkstoff Suramin in einer Konzentration von 100 bis 200 µg/ml umfaßt.

15. Produkt nach einem der Ansprüche 8 bis 14, zur Verwendung als kosmetisches, hygienisches oder Zahnpflegemittel oder -produkt.

16. Produkt nach einem der Ansprüche 8 bis 15, dadurch gekennzeichnet, daß es einen festen Träger umfaßt, auf den mittels eines beliebigen geeigneten Verfahrens der oder die Wirkstoffe aufgebracht sind.

17. Produkt nach Anspruch 16, dadurch gekennzeichnet, daß es aus Taschentüchern, Servietten, Bandagen, Verbänden, Handschuhen, Verpackungsmaterial, Preservativen, Diaphragmen, Watte, Toilettenpapier oder analogen Mitteln besteht.

18. Produkt nach einem der Ansprüche 8 bis 15, dadurch gekennzeichnet, daß es eine Masse umfaßt, mit der der oder die Wirkstoffe vermischt sind.

19. Produkt nach Anspruch 18, dadurch gekennzeichnet, daß es aus Zahnpasta, Salbe, Creme, Shampoo oder Schminke besteht.

20. Produkt nach einem der Ansprüche 8 bis 15, dadurch gekennzeichnet, daß es einen flüssigen Träger umfaßt, in dem der oder die Wirkstoffe in Lösung oder Suspension vorliegen.

21. Produkt nach Anspruch 20, dadurch gekennzeichnet, daß es aus einem Mundspülmittel, einer Sprühlösung, einem Deodorant oder einer Lotion besteht.

22. Verwendung eines Wirkstoffes, ausgewählt aus Benzidin, Dianilidharnstoff, Suramin, Pyridium, Neotropin und aromatischen Diazoverbindungen, wie Kongorot, Trypanblau, Trypanrot und Trypanviolett, für die Herstellung eines prophylaktischen Arzneimittels, das für das lokale Eindringen beim Kontakt mit Haut, Schleimhäuten oder Körperabsonderungen und zur Hemmung der Ausbreitung an dieser Stelle eines Virus aus der Gruppe der Retroviren, insbesondere des menschlichen Immunschwächevirus HIV, bestimmt ist.
